# EUROPEAN PATENT APPLICATION

(11) **EP 1 688 162 A1**
(43) Date of publication of application: **09.08.2006**
(21) Application number: 05388007.6
(22) Date of filing: 28.01.2005
(51) Int. Cl.: A61Q 11/00, A61K 8/19, A61K 8/21, A61K 8/24, A61K 8/34, A61K 8/97

(54) **Multi functional oral care chewing gum possessing tooth cleaning effects**

(71) Applicant: Gumlink A/S, 7100 Vejle (DK)
(72) Inventor: Kristiansen, Tove Nordestgaard, 7300 Jelling (DK); Gyldenvang, Lars, 8500-Grenaa (DK); Mikkelsen, Rikke, 7100 Vejle (DK)
(74) Representative: Indahl, Peter Jensen

(57) **Abstract**

A chewing gum possessing tooth cleaning effects, which chewing gum includes at least gum base and: a) at least one active cosmetic ingredient selected from whitening agents, b) at least one active cosmetic ingredient selected from fresh-breath agents, c) at least one active therapeutic ingredient selected from anti-plaque agents, d) at least one active therapeutic ingredient selected from anti-gingivitis agents. The chewing gum is intended for round the year daily use as the major tooth cleaning agent.

## Description

The present invention relates to a chewing gum possessing tooth cleaning effects.

Chewing gum suitable for temporary tooth cleaning is well-known. Such chewing gum is e.g. disclosed in the patent documents US 5,380,530, US 5,693,334, US 6,365,130 B1 and US 2004/0115247 A1. However, the known types of chewing gum has hitherto only been capable to demonstrate a limited capacity for tooth cleaning for single day use or at the most a few days use when a toothbrush is accidentally not available. After such period a thorough cleaning with toothbrush and toothpaste has been required. The known types of chewing gum with tooth cleaning effect have also been used as a supplement to daily cleaning with a toothbrush and toothpaste.

Brushing of teeth with a toothbrush is, however, rather rough on the teeth and especially on the gingiva and will eventually unavoidable lead to abrasive damage on the treated teeth or on the gingiva.

For several decades professionals and also in general adults and particularly parents have been convinced that the only suitable method of properly cleaning teeth is daily use of toothbrush and toothpaste.

Most people experience now and then that a toothbrush is unavailable and then have to resort to using other means, such as a chewing gum or lozenges, or using gargle, in order to at least obtain a sensation of fresh breath. But they are clearly aware that this does not account to proper tooth cleaning. People continue to brush teeth daily even when they experience brushing or abrasive damages. They will sometimes change to use toothbrushes having softer brushes or use electrical toothbrushes in order to obtain more favourable brush movements over the teeth and gingiva surfaces, but they do not dispense with tooth brushing as such.

An object of the present invention is to provide a dental care product that can be used daily without any risk of causing abrasive damage on the gingiva.

Consequently, the present invention relates to a chewing gum possessing tooth cleaning effects, which chewing gum includes at least gum base and:
a) at least one active cosmetic ingredient selected from whitening agents,
b) at least one active cosmetic ingredient selected from fresh-breath agents,
c) at least one active therapeutic ingredient selected from anti-plaque agents,
d) at least one active therapeutic ingredient selected from anti-gingivitis agents,
e) optionally at least one active therapeutic ingredient selected from re-mineralization agents, and
f) optionally at least one active therapeutic ingredient selected from anti-calculus agents.

The chewing gum with the active whitening agent(s), the active fresh-breath agent(s), the active anti-plaque agent(s), and the active anti-gingivitis agent(s) provides tooth cleaning effects of an efficiency that allows the user to clean the teeth by chewing the chewing gum instead of using a conventional toothbrush. Chewing of the chewing gum according to the invention does not cause abrasive damage on the gingiva.

Optionally the chewing gum may further include at least one active therapeutic ingredient selected from re-mineralization agents, and/or at least one active therapeutic ingredient selected from anti-calculus agents in order to enhance the tooth cleaning effects of the chewing gum.

The present invention actually provides a surprising solution to the existing problem of how to clean teeth and at the same time avoid the highly undesired effect of causing abrasive damage on the teeth and gingiva. The present invention makes it possible to replace daily tooth brushing by chewing of chewing gum, thus making teeth cleaning with toothbrush and toothpaste superfluous. As a consequence the highly undesired effect of abrasive damage on the teeth and gingiva is eliminated, while the cleaning quality of the teeth substantially corresponds to the cleaning quality obtained by toothbrush and toothpaste.

With the present invention it has unexpected been realised that a chewing gum according to the invention can be used as a tooth cleaning agent for daily use.

The chewing gum according to the invention is much easier and more convenient in use than the conventional toothbrush with toothpaste. This is in particular advantageously in case of children and disabled persons to whom handling of a toothbrush can be extremely difficult, which fact may very easily lead to defective teeth cleaning. Moreover, the chewing gum according to the invention can be used anywhere at any desired time, as there is no need for access to water, like e.g. in a bathroom. The chewing gum according to the invention can e.g. be used when driving a car, during work, while watching television etc, thereby providing much more freedom to the user.

It is preferred that the chewing gum, when chewed on a daily basis as a tooth cleaning agent, provides
a) a whitening effect corresponding to at least 50% of the whitening effect of daily brushing of teeth with a new toothbrush,
b) a fresh-breath effect corresponding to at least 100% of the fresh-breath effect of daily brushing of teeth with a new toothbrush,
c) an anti-plaque effect corresponding to at least 60% of the anti-plaque effect of daily brushing of teeth with a new toothbrush,
d) an anti-gingivitis effect corresponding to at least 65% of the anti-gingivitis effect of daily brushing of teeth with a new toothbrush,
e) optionally a re-mineralization effect corresponding to at least 65% of the re-mineralization effect of daily brushing of teeth with a new toothbrush,
f) optionally an anti-calculus effect corresponding to at least 25% of the anti-calculus effect of daily brushing of teeth with a new toothbrush, and
g) a substantially lower abrasive effect than the abrasive effect caused by daily brushing of teeth with a new toothbrush.

A new toothbrush has good properties in respect of tooth cleaning, but a toothbrush will unavoidable cause abrasive damage at least to some degree. However, during use the toothbrush will gradually lose its tooth cleaning properties, but still be able to cause abrasive damage. Consequently, in a relative short time the toothbrush cleaning properties in respect of whitening, plaque and gingivitis will decrease to a level of about 50-65% compared to the properties for a new toothbrush. The chewing gum according to the invention is in its nature always new, because a new piece of chewing gum is used each time the teeth are cleaned by chewing the gum. The tooth cleaning obtained by chewing the gum may perform significantly better than a used toothbrush. As most people do not change toothbrush every day, but commonly use the same toothbrush for a month or more, the cleaning properties of such a brush are not optimal.

In a preferred embodiment of the chewing gum according to the invention the whitening effect corresponds to at least 65%, preferably at least 85%, and more preferably at least 100%, of the whitening effect of daily brushing of teeth with a new toothbrush.

With the aim of obtaining the best possible tooth cleaning result with the chewing gum according to the invention the following embodiments are preferred.

In a preferred embodiment of the chewing gum according to the invention the anti-plaque effect corresponds to at least 65%, preferably at least 85%, and more preferably at least 100%, of the anti-plague effect of daily brushing of teeth with a new toothbrush.

In a preferred embodiment of the chewing gum according to the invention the anti-gingivitis effect corresponds to at least 75%, preferably at least 85%, and more preferably at least 100%, of the anti-gingivitis effect of daily brushing of teeth with a new toothbrush.

In a preferred embodiment of the chewing gum according to the invention the re-mineralization effect corresponds to at least 75%, preferably at least 85%, and more preferably at least 100%, of the re-mineralization effect of daily brushing of teeth with a new toothbrush.

In a preferred embodiment of the chewing gum according to the invention the anti-calculus effect corresponds to at least 50%, preferably at least 65%, and more preferably at least 100%, of the anti-calculus effect of daily brushing of teeth with a new toothbrush.

It is estimated that that the overall effect of chewing gum for dental cleaning compared to brushing teeth can be set to at least 70% relative to brushing teeth. This estimate is based on correct use of a new toothbrush (2 minutes brushing by a skilled user) compared to chewing of chewing gum for 5 to 20 minutes. Depending on the number of active ingredients in the chewing gum according to the present invention the overall effect of chewing gum for cleaning of teeth may exceed 100%.

In order to obtain a satisfactory cleaning effect of the chewing gum according to the invention it is preferred that the chewing gum is chewed at least once per day, and preferably two or more times per day (needless to say that for every new period of chewing a fresh piece of chewing gum should be used). In respect of the same issue of satisfactory cleaning it is preferred that each piece of the chewing gum is chewed for about 5 to about 20 minutes, such as longer than 10 minutes. Chewing for more than 5 minutes allows active ingredients in the chewing gum to be released and be given time to affect the teeth and gingiva. The break down of plaque, calculus, etc. by active ingredients is based on chemical reactions rather than on a mechanical rubbing effect and the longer duration causes improved effects.

In an embodiment of the chewing gum according to the invention the chewing gum is manufactured from traditional coherent gum. In this manner the active agents and optionally other ingredients are mixed into the gum base mass. The mixing operation may take place at elevated temperature to decrease the viscosity of the chewing gum formulation thereby facilitating the mixing. After the mixing the chewing gum formulation is normally sent through rollers to form sheets of chewing gum from which pieces of chewing gum are punched out.

In another embodiment the chewing gum is manufactured from compressed granules. Thus, the gum base is present as granules and is mixed with the active agents, which may also be present as granules or powder and optionally other ingredients. The mixture is filled into a press that presses the mixture to form compressed chewing gum tablets. Use of granules is particular advantageously when one ore more of the active ingredients are sensitive towards elevated temperatures as the mixing and pressing can be done at low temperature, e.g. normal room temperature.

In a preferred embodiment of the chewing gum according to the invention the chewing gum is layered. The chewing gum may comprise two or three or more layers. The layers are preferably placed on top of each other but can also be placed side by side or one inside the other. Optionally the layers have different colours.

When the chewing gum is layered it is possible to provide embodiments wherein different active therapeutic ingredients are present in different layers in the chewing gum. This is particular advantageously when the active therapeutic agents are mutual reactive. When the agents are present in different layers undesired reactions between the agents might be avoided.

Similar conditions apply in corresponding embodiments where different active cosmetic ingredients are present in different layers in the chewing gum.

For some embodiments of the chewing gum it is preferred that the chewing gum is coated. A coating may protect the active agents from decomposition e.g. caused by oxygen. Moreover, a coating may contribute to maintain a desired moisture content in the chewing gum or other physical conditions required to avoid break down of an active ingredient. The coating may be a hard coating or a film coating.

Accordingly a chewing gum according to the invention, wherein at least one active therapeutic agent and/or at least one active cosmetic agent are present in the coating may be provided. Such an embodiment may for instance be advantageously when a rapid release of one or more active agents is desirable.

The invention also encompasses an embodiment, in which the chewing gum comprises at least one barrier layer. A barrier layer may serve to separate two active agents that will react when mixed. Optionally the barrier layer is a layer in a layered tablet e.g. a chewing gum tablet comprising three or more layers.

According to the invention it is preferred that the one or more anti-plaque agents constitute(s) 0.01-70% (high content of anti-plaque agent is in particular desirable when the anti-plaque agent is xylitol), preferably 0.03-50%, more preferred 0.05-35% of the chewing gum.

Preferably the one or more anti-gingivitis agents constitute(s) 0.01-20%, more preferred 0.03-12% of the chewing gum.

Preferably the one or more anti-calculus agents constitute(s) 0.01-20%, more preferred 0.03-15% of the chewing gum.

Preferably the one or more re-mineralization agents constitute(s) 0.01-20%, more preferred 0.02-10 of the chewing gum.

Preferably the one or more whitening agents constitute(s) 0.01-20%, preferably 0.03-12% of the chewing gum.

Preferably the one or more fresh-breath agents constitute(s) 0.01-20%, more preferred 0.02-8% of the chewing gum.

The above listed ranges for content of active ingredients provide effective amounts of active therapeutic and cosmetic ingredients. The total amount of active ingredients should preferably, not exceed 35% (with the exception of xylitol, which may constitute up to 70% of the chewing gum) based on the total weight of the chewing gum. Preferably the active therapeutic ingredients constitutes about 5 to 25%, and the active cosmetic ingredients constitutes about 2 to 15% of the chewing gum based on the total weight of the chewing gum.

In a preferred embodiment of the chewing gum according to the invention the gum base further includes at least one anti-bacterial agent, preferably selected from xylitol, chlorhexidine, neem, green tea, thyme, and Icelandic moss, and the antibacterial agent preferably constitutes about 0.4 to 7.5% of the chewing gum.

For the purpose of securing a non-damaging abrasive effect of the chewing gum according to the invention it is preferred that the chewing gum is substantially free of abrasives. However, the chewing gum may contain minor amounts of abrasive and polishing agents, in particular if these are softer than dental enamel and dentine. Such abrasives and polishing agents may serve to mechanically break down plaque and calculus.

As some people are allergic or suffer from other conditions caused by metal ions, it is preferred that the anti-calculus agents and/or the anti-gingivitis agents and/or the re-mineralization agents in the chewing gum are free from metal ions.

Although two or more of the whitening agents, fresh-breath agents, anti-plaque agents, and anti-gingivitis agents may be constituted by the same agent, it is for most embodiments of the chewing gum according to the invention preferred that the at least one whitening agent, the at least one fresh-breath agent, the at least one anti-plaque agent, and the at least one anti-gingivitis agent are mutually different substances. Such embodiments will normally provide the best tooth cleaning effects.

Preferably the chewing gum according to the invention is being intended for round the year daily use as the major tooth cleaning agent.

Moreover, in a further aspect the invention relates to use of a chewing gum according to the invention as the major tooth cleaning agent for a period of at least one year.

The invention also relates to use of a chewing gum according to the invention as a substitute to tooth brushing for a period of at least three months.

In this manner the chewing gum may be used as a tooth cleaning agent by people who for some reason or another (e.g. due to abrasive damage on the teeth) are unable to use a toothbrush for tooth cleaning.

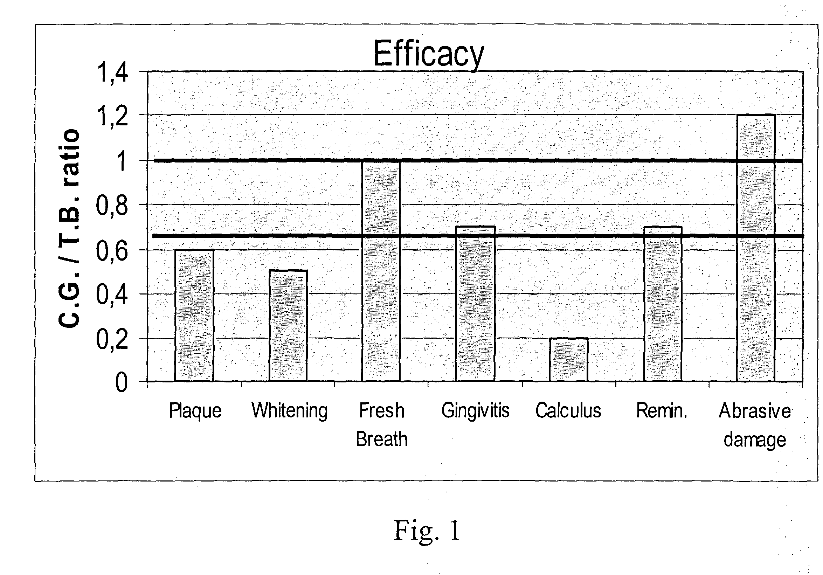

The invention will now be further illustrated with reference to figure 1 and examples.

Gum base refers in general to any commercially available gum base suitable for production of chewing gum. Such gum bases are well-known and available in the market and normally comprise natural and/or synthetic resins and optionally other ingredients. The gum base may be biodegradable, and such gum bases are also commercially available.

Chewing gum is the final product, including gum base, active ingredients and optional other ingredients such as taste ingredients and colouring agents. The chewing gum is ready to use by the consumer for cleaning teeth.

Active therapeutic ingredient means any ingredient that by itself has a therapeutic effect on the teeth and the oral environment including gingiva. Some active therapeutic ingredients may be active against more than one condition, e.g. function as both anti-plaque agent and anti-calculus agent, and they are in the present context listed under both functions.

Active cosmetic ingredients are ingredients that have cosmetic effect on the teeth and oral cavity, i.e. improve the appearance of the teeth including odour.

All percentages (%) are percentage by weight unless otherwise stated.

The chewing gum according to the invention can be conventional chewing gum pieces, compressed chewing gum tablets, sticks, centre-filled chewing gum with the centre filled with liquid, gel or powder. Moreover, the active ingredients, flavour and sweetener may be encapsulated to avoid undesired reactions during storage.

Figure 1 illustrates an estimate of the efficacy of the chewing gums tested (C.G)(chewing for 5 to 20 minutes) for dental care purposes compared with tooth brushing (T.B)(new tooth brush and correct tooth brushing for 2 minutes).

The line at 1 indicates the efficacy of a toothbrush and the columns indicates the efficacy of chewing gum in respect of plaque, whitening, fresh breath, gingivitis, calculus, re-mineralization and abrasive damage, respectively. As indicated by the line, the overall efficacy of tested chewing gum compared to tooth brushing is about 67%. The individual effects can be improved by adding more active ingredients and/or by combining ingredients so that the efficacy is raised above 70%, such as an efficacy that exceeds 100% or better efficacy than tooth brushing.

In respect of plaque (efficacy approx 60% for the tested chewing gum) the removal of plaque and/or inhibition of plaque formation can been improved, e.g. by adding zinc acetate to the chewing gum, which will enhance the effect to be close to or better than the efficacy for brushing teeth. In addition, the chewing gum according to the invention will be better than brushing teeth in real life due to the fact that the effect from chewing gum reaches places the toothbrush cannot reach. An in vivo plaque study performed by the inventors shows that e.g. zinc acetate worked in the "hard-to-reach" places with high efficiency.

Moreover, a clinical test has demonstrated that chewing gum with calcium pyrophosphate clinically whitens teeth. Presently, the effect almost matches the effect of a toothbrush with toothpaste. However, by using other agents the whitening effect will reach 100% as compared with tooth brushing.

The fresh breath effect is already better than if brushing teeth, as the chewing gum according to the invention has a much longer contact time with the volatile sulphur compounds to be eliminated. Correct tooth brushing last 2 minutes whereas chewing of chewing gum last for at least 5 minutes.

With respect of the anti-gingivitis effect, chewing gum according to the invention already match this effect compared to brushing of teeth, as the contact time is longer as with brushing teeth, thereby allowing the active substances longer time to affect the infected gingiva.

As chewing gum stimulates saliva, and a wide range of active ingredients can be added to the chewing gum according to the invention that promotes re-mineralization or alternatively inhibits demineralization, the re-mineralization effect of the chewing gum exceeds the effect of brushing of teeth.

Chewing gum substantially have no abrasive effect on teeth and this parameter is naturally dramatically better than brushing teeth.

### Example

The chewing gum in the following example was manufactured from commercially available gum base (Danfree, available from Gumlink A/S, Vejle, Denmark) mixed with sweeteners, taste ingredients and active ingredients. The chewing gum was manufactured as a two-layer product and the gum material for the two layers was produced with the following ratios:

| Formulation 1: | |
|---|---|
| Gum base | 60.00% |
| Sorbitol | 18.20% |
| Peppermint powder | 1.50% |
| Menthol powder | 0.30% |
| Dicalciumphosphate | 2.70% |
| Green tea | 5.00% |
| Baking soda | 0.40% |
| Calcium carbonate | 4.10% |
| Calcium pyrophosphate | 6.50% |
| Succralose | 0.25% |
| Magnesium stearate | 0.50% |
| Eucalyptus powder | 0.50% |

| Formulation 2: | |
|---|---|
| Gum base | 30.00% |
| Xylitol. | 68.80% |
| Peppermint powder | 0.50% |
| Menthole powder | 0.20% |
| Magnesium stearate | 0.50% |

The gum base was granulated (GALA underwater pelletizer) to form granules with diameters in the range of approximately 0.5 - 1.5 mm and mixed with the active ingredients.

The particulate mixture of formulation 1 (1.5 g) was filled into a tablet pressing machine and compressed to form a first layer. Then 0.7 g pure gum base granules were filled into the tablet pressing machine and compressed onto the first layer to form a barriere layer. Finally 2 g of formulation 2 particulate material was filed into the tablet pressing machine and compressed.

The resulting cylindrical shaped layered chewing gum tablets had an average weight of about 4.2 g and a diameter of about 8 mm.

The chewing gum was evaluated for inhibition of plaque formation in a clinical study.

The test subjects abstained from all oral hygiene for 2 days and either chewed the gum five times per day or used no gum (Plaque scores were assigned using the Modified Quickly-Hein (MQH) index). The result demonstrated that chewing gum comprising xylitol was significantly more effective in inhibiting the formation of plaque on teeth when used as the only means of oral hygiene for two days. Additionally, it was most efficient in areas that are often missed during tooth brushing.

In conclusion, the results demonstrate that the chewing gum containing xylitol is able to reduce dental plaque formation. Moreover, the chewing gum has an ability to make dental plaque less adhesive and thus easier to remove during chewing. As a further benefit, xylitol inhibit bacterial growth and thereby inhibit tooth decay.

The chewing gum was also evaluated for its whitening effect. The chewing gum comprising calcium pyrophosphate not only results in whiter teeth by stain removal, it also helps to prevent further stain after consumption of foods and beverages.

Clinical studies on the inhibition of stain over a 14 days period showed that when chewing, chewing gum according to the invention 20 minutes each day, compared to chewing, chewing gum with 4.5% calcium carbonate, commercially available on the market, the inhibition of stain was considerably improved.

The dicalcium phosphate in the chewing gum improves the re-mineralization rate of the teeth.

Green tea provided excellent fresh breath properties in the chewing gum.

The chewing gum according to the invention is preferably a chewing gum wherein at least 55% of the active therapeutic ingredients are released after 5 minutes of chewing when measured according to Ph. Eur. (European Pharmacopoeia) Version 5.0, 01/2005, paragraph 2.9.25 (volume 1 page 260). Preferably at least 75% of the active therapeutic ingredients are released after 15 minutes of chewing when measured according to Ph. Eur. Version 5.0, 01/2005, paragraph 2.9.25 (volume 1 page 260). In this embodiment the amounts of the active therapeutic ingredients are released within the preferred chewing time of 5 to 20 minutes.

Moreover, the chewing gum according to the invention is preferably a chewing gum wherein at least 30% of the active cosmetic ingredients are released after 5 minutes of chewing when measured according to Ph. Eur. Version 5.0, 01/2005, paragraph 2.9.25 (volume 1 page 260). Preferably at least 50% of the active cosmetic ingredients are released after 10 minutes of chewing when measured according to Ph. Eur. Version 5.0, 01/2005, paragraph 2.9.25 (volume 1 page 260). This preferred embodiment provides for release of the active cosmetic ingredients within the preferred chewing time of 5 to 20 minutes.

In embodiments where the chewing gum is provided with a hard coating, the hard coating can be a coating selected from the group consisting of a sugar coating and a sugarless coating and a combination thereof.

In a further embodiment of the invention the hard coating comprises 50 to 100% by weight of a polyol typically selected from the group consisting of sorbitol, maltitol, mannitol, xylitol, erythritol, lactitol and isomalt.

In an alternative embodiment of the invention the outer coating is an edible film comprising at least one component selected from the group consisting of an edible film-forming agent and a wax. In a preferred embodiment of the invention the film-forming agent is selected from the group consisting of a cellulose derivative, a modified starch, a dextrin, gelatine, shellac, gum arabic, zein, a vegetable gum, a synthetic polymer and any combination thereof.

In an embodiment of the invention the outer coating comprises at least one additive component selected from the group consisting of a binding agent, a moisture absorbing component, a film forming agent, a dispersing agent, an anti-sticking component, a bulking agent, a flavouring agent, a colouring agent, a pharmaceutically or cosmetically active component, a lipid component, a wax component, a sugar, an acid and an agent capable of accelerating the after-chewing degradation of the degradable polymer.

In an embodiment the outer coating is a soft coating comprising a sugar free coating agent.

The invention also encompasses an embodiment, in which the chewing gum comprises at least one barrier layer. A barrier layer may serve to separate two active agents that will react when mixed. Optionally the barrier layer is a layer in a layered tablet e.g. a chewing gum tablet comprising three or more layers.

In the following further examples of ingredients are mentioned.

Anti-plaque agents include fluoride ion sources. Anti-plaque agents are any substance which by itself acts to inhibit the accumulation of bacterial deposits on the surfaces of the oral cavity. Examples include xylitol and other anti-microbial agents. The inhibition effects of the xylitol on oral microbes may have better effect when used in conjunction with an extract since the extract is also acting to disable the microbes.

Typical examples of active ingredients that are particularly desirable from considerations of anti-plaque effectiveness, safety and formulation are:
Naficillin, oxacillin, vancomycin, clindamycin, erythromycin, trimethoprim-sulphamethoxazole, rifampin, ciprofloxacin, broad spectrum penicillin, amoxicillin, gentamicin, ceftriazoxone, cefotaxime, chloramphenicol, clavunate, sulbactam, probenecid, doxycycline, spectinomycin, cefixime, penicillin G, minocycline, .beta.-lactamase inhibitors; meziocillin, piperacillin, aztreonam, norfloxacin, trimethoprim, ceftazidime, dapsone. Halogenated diphenyl ethers, e.g. 2',4,4'-trichloro-2-hydroxydiphenyl ether (Triclosan), 2,2'-dihydroxy-5,5'-dibromo-diphenyl ether. Haloqenated salicylanilides, e.g. 4',5-dibromosalicylanilide, 3,4',5-trichloro-salicylanilide, 3,4',5-tribromo-salicylanilide, 2,3,3',5-tetrachloro-salicylanilide, 3,3,3',5-tetrachloro-salicylanilide, 3,5-dibromo-3'-trifluoromethyl-salicylanilide, 5-n-octanoyl-3'-trifluoromethyl-salicylanilide, 3,5-dibromo-4'-trifluoromethyl-salicylanilide, 3,5-dibromo-3'-trifluoromethyl-salicylanilide (Flurophene). Benzoic esters, e.g. methyl-p-hydroxybenzoic ester, ethyl-p-hydroxybenzoic ester, propyl-p-hydroxybenzoic ester, butyl-p-hydroxybenzoic ester. Halogenated carbanilides, e.g. 3,4,4'-trichlorocarbanilide, 3-trifluoromethyl-4,4'-dichlorocarbanilide, or 3,3,4' - trichlorocarbanilide. Phenolic compounds (including phenol and its homologs, mono- and poly-alkyl and aromatic halo-phenol and their homologs), e.g. phenol, 2-methyl-phenol, 3-methyl-phenol, 4-methyl-phenol, 4-ethyl-phenol, 2,4-dimethyl-phenol, 2,5-dimethyl-phenol, 3,4-dimethyl-phenol, 2,6-dimethyl-phenol, 4-n-propyl-phenol, 4-n-butyl-phenol, 4-n-amyl-phenol, 4-tert-amyl-phenol, 4-n-hexyl-phenol, 4-n-heptyl-phenol, 2-methoxy-4-(2-propenyl)-phenol (Eugenol), 2-isopropyl-5-methyl-phenol (Thymol), mono- and poly-alkyl- and aralkyl-halophenols, methyl-p-chlorophenol, ethyl-p-chlorphenol, n-propyl-p-chlorophenol, n-butyl-p-chlorophenol, n-amyl-p-chlorophenol, sec-amyl-p-chlorophenol, n-hexyl-p-chlorophenol, cyclohexyl-p-chlorophenol, n-heptyl-p-chlorophenol, n-octyl-p-chlorophenol, o-chlorophenol, methyl-o-chlorophenol, ethyl-o-chlorophenol, n-propyl-o-chlorophenol, n-butyl-o-chlorophenol, n-amyl-o-chlorophenol, tert-amyl-o-chlorophenol, n-hexyl-o-chlorophenol, n-heptyl-o-chloropenol, p-chlorophenol, o-benzyl-p-chlorophenol, o-benzyl-m-methyl-p-chlorophenol, o-benzyl-m,m-dimethyl-p-chlorophenol, o-phenylethyl-p-chlorophenol, o-phenylethyl-m-methyl-p-chlorophenol, 3-methyl-p-chlorophenol, 3,5-dimethyl-p-chlorophenol, 6-ethyl-3-methyl-p-chlorophenol, 6-n-propyl-3-methyl-p-chlorophenol, 6-iso-propyl-3-methyl-p-chlorophenol, 2-ethyl-3,5-dimethyl-p-chlorophenol, 6-sec-butyl-3-methyl-p-chlorophenol, 2-iso-propyl-3,5-dimethyl-p-chlorophenol, 6-diethylmethyl-3-methyl-p-chlorophenol, 6-iso-propyl-2-ethyl-3-methyl-p-chlorophenol, 2-sec-amyl-3,5-dimethyl-p-chlorophenol, 2-diethylmethyl-3,5-dimethyl-p-chlorophenol, 6-sec-octyl-3-methyl-p-chlorophenol, p-bromophenol, methyl-p-bromophenol, ethyl-p-bromophenol, n-propyl-p-bromophenol, n-butyl-p-bromophenol, n-amyl-p-bromophenol, sec-amyl-p-bromophenol, n-hexyl-p-bromophenol, cyclohexyl-p-bromophenol, o-bromophenol, tert-amyl-o-bromophenol, n-hexyl-o-bromophenol, n-propyl-m,m-dimethyl-o-bromophenol, 2-phenyl-phenol, 4-chloro-2-methyl-phenol, 4-chloro-3-methyl-phenol, 4-chloro-3,5-dimethyl-phenol, 2,4-dichloro-3,5-dimethyl-phenol, 3,4,5,6-tetrabromo-2-methylphenol, 5-methyl-2-pentylphenol 4-isopropyl-3-methylphenol 5-chloro-2-hydroxydiphenyl-methane. Resorcinol and its derivatives, e.g. resorcinol, methyl-resorcinol, ethyl-resorcinol, n-propyl-resorcinol, n-butyl-resorcinol, n-amyl-resorcinol, n-hexyl-resorcinol, n-heptyl-resorcinol, n-octyl-resorcinol, n-nonyl-resorcinol, phenyl-resorcinol, benzyl-resorcinol, phenylethyl-resorcinol, phenylpropyl-resorcinol, p-chlorobenzyl-resorcinol, 5-chloro-2,4-dihydroxydiphenyl-methane, 4'-chloro-2,4-dihydroxydiphenyl-methane, 5-bromo-2,4-dihydroxydiphenyl-methane, 4"-bromo-2,4-dihydroxydiphenyl-methane. Bisphenolic compounds, e.g. bisphenol A, 2,2'-methylene-bis-(4-chlorophenol), 2,2'-methylene-bis-(3,4,6-trichlorophenol) (hexachlorophene), 2,2'-methylene-bis-(4-chloro-6-bromophenol), bis-(2-hydroxy-3,5-dichlorophenyl)-sulfide, bis-(2-hydroxy-5-chlorobenzyl)-sulfide.

Illustrative of polyphosphate compounds with plaque-inhibiting properties are dialkali metal and tetraalkali metal pyrophosphate and mixtures thereof in a hydrated or unhydrated form. Illustrative of pyrophosphate salts are Na₂H₂P₂O₇, Na₄P₂O₇ and K₄P₂O₇. Other suitable polyphosphates include hydrated or unhydrated alkali metal tripolyphosphates such as Na₅P₃O₁₀ and K₅P₃O₁₀.

Plaque buffers such as urea, calcium lactate, calcium glycerophosphate and strontium polyacrylates, ammonium carbonate and vitamins such,as Vitamins A, C and E are also included.

Nutraceuticals and nutritional supplements may also be added to chewing gums as active agents against plaque. Among these are herbs and botanicals that include, but are not limited to chamomile, echinacea, Eucalyptus and green tea.

Metal cations can also be used as anti-bacterial and anti-plaque agents. The metal cations are selected from the metals of Group 5 (V, Nb, Ta); Group 6 (Cr, Mo, W); Group 7 (Mn, Tc, Re); Group 8 (Fe, Ru, Os); Group 9 (Co, Rh, Ir); Group 10 (Ni, Pd, Pt); Group 11 (Cu, Ag, Au); Group 12 (Zn, Cd, Hg); Group 14 (Ge, Sn, Pb); Group 16 (Se, Te, PO); and mixtures thereof. Preferably the metal cation is selected from any monovalent or divalent cation selected from the group consisting of zinc, manganese, copper, iron, cobalt, silver, selenium, tin and vanadium; preferably from the group consisting of zinc, manganese, copper, iron, silver, and tin; more preferably from the group consisting of zinc, copper, silver and tin and most preferably from the group consisting of zinc and tin.

Illustrative of zinc compounds with plaque-inhibiting properties are zinc oxide, zinc silicate, zinc carbonate, zinc phosphate, zinc stannate, zinc tetrafluoroborate, zinc hexafluorosilicate, zinc citrate, zinc benzoate, zinc oxalate, zinc stearate, zinc chloride, zinc sulfate, zinc nitrate, zinc phenolsulfonate, zinc carboxymethylsuccinate, and the like. The zinc compound also can be in the form of a complex, with a complexing reagent such as polyethylenimine or ethylenediamine tetraacetic acid.

A wide variety of metal cation salts are useful in the present invention. These include so called "water-insoluble salts" which have a solubility of less than about 0.5 g per 100 ml at 25 degree C and "water soluble salts" which have a solubility of greater than or equal to about 0.5 g per 100 ml at 25 degree C. It is also possible to use mixtures of these salts. Such mixtures can have several advantages in the compositions of the present invention since they are likely to have different complexing properties with the polyphosphate anions. In addition they have different release rates in the saliva and can therefore act to provide controlled release profiles. Examples of salts that are suitable for use herein include acetate, ammonium sulphate, bromide, chloride, chromate, citrate, dithionate, fluorosilicate, tartrate, fluoride, formate, iodide, nitrate, phenol sulphate, salicyclate, sulphate, gluconate, succinate, glycerophosphate, lactate and mixtures thereof.

Anti-gingivitis agents can be antiinflammatory agents, such as salicylic acid derivatives (e.g. aspirin), paraminophenol derivative (e.g. acetaminophen), indole and indene acetic acids (indo-methacin, sulindac and etodalac), heteroaryl acetic acids (tolmetin, diclofenac and ketorolac), aryl propionic acid derivatives (ibuprofen, naproxen, ketoprofen, fenopren, oxaprozine), anthranilic acids-(mefenamic acid, meclofenamic acid), enolic acids (piroxicam, tenoxicam, phenylbutazone and oxyphenthatrazone), lactic acid bacteria (LAB), Osteopontin (ONP), IG-Lyt, hexefine, Aloe Vera, chlorhexedine, myrrh, or sage.

Anti-gingivitis agents also comprise psychotherapeutic agents, such as thorazine, serentil, mellaril, millazine, tindal, permitil, prolixin, trilafon, stelazine, suprazine, taractan, navan, clozaril, haldol, halperon, loxitane, moban, orap, risperdal, alprazolam, chlordiaepoxide, clonezepam, clorezepate, diazepam, halazepam, lorazepam, oxazepam, prazepam, buspirone, elvavil, anafranil, adapin, sinequan, tofranil, surmontil, asendin, norpramin, pertofrane, ludiomil, pamelor, vivactil, prozac, luvox, paxil, zoloft, effexor, welibutrin, serzone, desyrel, nardil, parnate, or eldepryl.

Anti-calculus agents suitable for use in the chewing gum according to the invention include phosphates, pyrophosphates, alkali-metal pyrophosphates, polyphosphates, phosphonates, polyphosphonates and mixtures of any of these. Pyrophosphates are among the best known for use in dental care products. The pyrophosphate salts useful in the present invention include the di-alkali metal pyrophosphate salts, tetra-alkali metal pyrophosphate salts and mixtures of any of these in their unhydrated as well as hydrated forms are the preferred species. Di-sodium dihydrogen pyrophosphate (Na₂H₂P₂O₇), tetra-sodium pyrophosphate (N₄P₂O₇), and tetrapotassium pyrophosphate (K₄P₂O₇) and mixtures thereof are specific examples.

Additional suitable anti-calculus agents include polyacrylates and other polycarboxylates, such as those disclosed in US Patent No. 3,429,963, US Patent No. 4,304,766, and US Patent No. 4,661,341, polyepoxysuccinates, such as those disclosed in US Patent No. 4,846,650, ethylenediaminetetraacetic acid as disclosed in British Patent No. 490,384, nitrilotriacetic acid and related compounds as disclosed in US Patent No. 3,678,154, polyphosphonates as disclosed in US Patent No. 3,737,533, US Patent No. 3,988,443, and US Patent No. 4,877,603.

The re-mineralisation agents are preferably pH adjusting agents, which may also be added to make the composition safe for oral tissues. These pH adjusting agents, or buffers, can be any material that is suitable to adjust the pH of the composition. Suitable materials include sodium bicarbonate, sodium phosphate, sodium hydroxide, ammonium hydroxide, potassium hydroxide, sodium stannate, triethanolamine, citric acid, hydrochloric acid, sodium citrate, calcium, fluoride, Phoscal, dicalcium phosphate, Osteopontin (ONP), monosodium phosphate, trisodium phosphate, sodium hydroxide, sodium carbonate, pectin, benzocaine, analgesics, sanguinarine extract, metronidazole, strontium chloride, potassium nitrate, carrageenan, cough and cold remedies, and the like.

The preferred active therapeutic ingredients listed above have all demonstrated good effect in improving teeth and oral health.

The preferred active cosmetic ingredients are listed below. The mentioned ingredients have all proven to provide excellent cosmetic effects.

The whitening agents are conveniently selected from teeth colour modifying substances that may be considered among the oral care actives useful in the chewing gum according to the invention. These substance are suitable for modifying the colour of the teeth to satisfy the consumer such as those listed in the CTFA Cosmetic Ingredient Handbook, 3.sup.rd Edition, Cosmetic and Fragrances Association Inc., Washington D.C. (1982), incorporated herein by reference. Specific examples include talc, mica, magnesium carbonate, calcium carbonate, calcium pyrophosphate, Baking soda, Icelandic moss, bamboo, sodium hexa-metaphosphate, magnesium silicate, aluminium magnesium carbonate, silica, titanium dioxide, zinc oxide, red iron oxide, brown iron oxide, yellow iron oxide, black iron oxide, ferric ammonium ferrocyanide, manganese violet, ultramarine, nylon powder, polyethylene powder, methacrylate powder, polystyrene powder, silk powder, crystalline cellulose, starch, titanated mica, iron oxide titanated mica, bismuth oxychloride, and mixtures thereof. Typical levels are from about 0.05% to about 20%, preferably from about 0.1% to about 15% and most preferably from about 0.25% to about 10%, by weight, of the composition.

Whitening agents for use herein may also comprise materials that remove or bleach intrinsic or extrinsic stains on or in tooth surfaces. Such substances are selected from the group consisting of the peroxides, metal chlorites, perborates, percarbonates, peroxyacids, persulphates, and combinations thereof. Suitable peroxide compounds include hydrogen peroxide, urea peroxide, calcium peroxide, carbamide peroxide and mixtures thereof. Suitable metal chlorites include calcium chlorite, barium chlorite, magnesium chlorite, lithium chlorite, sodium chlorite and potassium chlorite. Additional bleaching substances may be hypochlorite, and chlorine dioxide. A preferred percarbonate is sodium percarbonate. Preferred persulphates are oxones. The content of these substances is dependent on the available oxygen or chlorine. The content of these ingredients in the chewing gum according to the invention is generally in the range from about 0.1% to about 35%, preferably from about 1% to about 25% and most preferably from about 5% to about 10%, by weight of the chewing gum.

The fresh-breath agents are preferably selected from agent for oral malodour control, which include a wide variety of materials. Suitable in the chewing gum according to the invention are anti-microbial agents. Such agents may include 5-chloro-2-(2,4-dichlorophenoxy)-phenol, commonly referred to as triclosan, and described in the Merck Index, 11^{th} Edition, (1989), pp1529 (entry No. 9573) in US Patent No. 3,506,720, and in European Patent publication No. 0 251 591, phthalic acid and its salts including, but not limited to those disclosed in US Patent No. 4,994,262, preferably magnesium mono-potassium phthalate, chlorohexidine (Merck Index, No. 2090), alexidine (Merck Index, No. 222), hexetidine (Merck Index, No. 4624), sanguinarine (Merck Index, No. 8320), benzalkonium chloride (Merck Index, No. 1066), salicylanilide (Merck Index, No. 8299), domiphen bromide (Merck Index, No. 3411), cetylpyridinium chloride (CPC) (Merck Index, No. 2024), tetradecylpyridinium chloride (TPC), N-tetradecyl-4-ethylpyridinium chloride (TDEPC), octenifine, delmopinol, octapinol, and other piperidine derivatives, nicin preparations, zinc/stannous ion agents, antibiotics, such as augmentin, amoxicilline, tetracycline, doxycycline, hexadine, minocycline, and metronidazole, and analogues and salts of the above, methyl salicyclate, and mixtures of any of the above.

Illustrative zinc compounds with fresh breath properties for use as fresh-breath agents are zinc oxide, zinc silicate, zinc carbonate, zinc phosphate, zinc stannate, zinc tetrafluoroborate, zinc hexafluorosilicate, zinc citrate, zinc benzoate, zinc oxalate, zinc stearate, zinc chloride, zinc sulfate, zinc nitrate, zinc phenolsulfonate, zinc carboxymethylsuccinate, and the like. The zinc compounds may also be present as a complex, with a complexing agent such as polyethylenimine or ethylenediamine tetraacetic acid.

A further group of natural extracts which are useful for their oral malodour control benefits include extracts obtained from the tea (green tea, red tea, white tea and black tea), honey suckle, coriander, thyme, propolis, tea tree oil, barberry bark, champex® , sunphenon, applephenon, gold thread, magnolia plants or mixtures thereof. Extracts suitable for use in the present invention can be obtained from any part of the plant including the leaf, stem, bark, pulp, seed, flesh, juice, root and mixtures thereof. It is preferred that chewing gum according to the present invention comprise from about 0.01% to about 5%.

The following essential oils are also known to have anti-microbial activity and are therefore optionally used in chewing gum according to the present invention. These oils include thymol, geraniol, carvacrol, hinokitiol, eucalyptol, catechol (particularly 4-allyl catechol), and mixtures thereof.

Another class of oral malodour control agents include absorbents. These are used to absorb, adsorb, bind or otherwise complex the volatile oral malodour materials. Examples of such agents include talc, mushroom extract, zeolite, cyclodextrin, silica shell and mixtures thereof. Such materials are preferably used in a range from about 0.5% to about 10%, preferably from about 1% to about 5%, by weight of the chewing gum.

In a preferred embodiment of the chewing gum according to the invention the chewing gum further comprises one or more taste ingredients selected from sweeteners, high-potent sweeteners and flavours. The sweeteners may e.g. be sweeteners like sucrose, dextrose, dextrins, maltose, trehalose, D-tagatose, dried invert sugar, ribose, fructose, levulose, galactose, glucose, maltodextrin, polydextrose, isomalt, sorbitol, sorbitol syrup, mannitol, xylitol, hexa-resorcinol, maltitol, isomaltol, erythriol, lactitol, xylose, tagatose and hydrogenated starch hydrolysates (maltitol syrup). The high potent sweeteners includes the dipeptides aspartame, neotame and alitame; N-sulfonylamides such as saccharin including the salts thereof and acesulfam including the salts thereof; sulfamates such as cyclamate including the salts thereof; chlorinated sugar derivatives such as sucralose; Terpenoid glycosides such as Rebaudioside-A, Stevioside and Glyhyrrhizin; proteins such as thaumatin and monellin and Di-hydrochalcones.

A variety of one or more flavouring agents may be used. Flavouring agents suitable for use in the present invention include natural, natural-identical, and/or artificial flavouring substance, or mixtures thereof, in their solid and/or in their liquid state. The person skilled in the art will recognize that natural and artificial flavouring agents may be combined in any sensorially acceptable blends. Some examples of suitable tastes are peppermint, lemon, and orange.

When taste ingredients like sweeteners and flavours are used, these are normally admixed to the gum base or the active ingredients. Taste ingredients in the chewing gum stimulates the user to chew for a prolonged period of time, which again have the advantages that the active ingredients has a longer period to be released and affect teeth and gingival surfaces.

Consequently, the polishing material can be any material that does not abrade dental enamel and dentine. Typical materials include silica gels and precipitates, aluminas, phosphates, and mixtures thereof. Specific examples include dicalcium orthophosphate dihydrate, calcium pyrophosphate, Bamboo, tricalcium phosphate, hydrated alumina, beta calcium pyrophosphate, calcium carbonate, sodium polymetaphosphate, sodium hexametaphosphate, Calgen, Giltex, Quadrafos, Hagan phosphate, micromet, calcium phosphate dibasic, calcium monohydrogen phosphate, dicalcium orthophosphate secondary calcium phosphate, carbonic acid calcium salt, cacti, calcichew, calcidia, citrical, aragonite, calcite, valerite, aluminum oxide, alumina, silicon dioxide, silica, silicic anhydride, and resinous abrasive materials such as particulate condensation products of urea and formaldehyde and others such as disclosed in US Patent No. 3,070,510. Mixtures of polishing agents can also be used.

The silica polishing materials generally have an average particle size ranging between about 0.1 to about 30 microns; and preferably from about 5 to about 15 microns. The polishing agent can be precipitated silica or silica gels, such as the silica xerogels described in US Patent No. 3,538,230 or in US Patent No. 3,862,307. Preferred are the silica xeropgels marketed under the name "Syloid" by the W. R. Grace and Company, Davison Chemical Division. Also preferred are the precipitated silica materials such as those marketed by the J. M. Huber Corporation under the trade name "Zeodent", particularly the silica carrying the designation "Zeodent 119". The types of silica dental polishing agents useful in the chewing gum of the present invention are described in more details in US Patent No. 4,340,583. The polishing agents in the chewing gum according to the invention is generally present in the range from about 6% to about 70%, preferably from about 10% to about 50%, by weight of the chewing gum.

In some embodiments the anti-plaque agents include one or more of the following: zinc acetate, ammonium carbamate, eucalyptus, cranberry, xylitol, chlorhexidine, and baking soda.

In some embodiments the anti-gingivitis agents include one or more of the following: chlorhexidine, myrrh, neem, sage and quince.

In some embodiments the anti-calculus agents include one or more of the following: vitamin C, citric acid, and acetic acid.

In some embodiments the re-mineralization agents include one or more of the following: calcium, fluoride, and phoscal.

In some embodiments the whitening agents include one or more of the following: baking soda, Icelandic moss, bamboo, calcium pyrophosphate, and sodium hexa-metaphosphate.
In some embodiments the fresh-breath agents include zinc acetate, coriander, green tea, and/or thyme.

## Claims

1. A chewing gum possessing tooth cleaning effects, which chewing gum includes at least gum base and:
a) at least one active cosmetic ingredient selected from whitening agents,
b) at least one active cosmetic ingredient selected from fresh-breath agents,
c) at least one active therapeutic ingredient selected from anti-plaque agents,
d) at least one active therapeutic ingredient selected from anti-gingivitis agents,
e) optionally at least one active therapeutic ingredient selected from re-mineralization agents, and
f) optionally at least one active therapeutic ingredient selected from anti-calculus agents.

2. A chewing gum according to claim 1, wherein the chewing gum when chewed on a daily basis as a tooth cleaning agent provides
a) a whitening effect corresponding to at least 50% of the whitening effect of daily brushing of teeth with a new toothbrush,
b) a fresh-breath effect corresponding to at least 100% of the fresh-breath effect of daily brushing of teeth with a new toothbrush,
c) an anti-plaque effect corresponding to at least 60% of the anti-plaque effect of daily brushing of teeth with a new toothbrush,
d) an anti-gingivitis effect corresponding to at least 65% of the anti-gingivitis effect of daily brushing of teeth with a new toothbrush,
e) optionally a re-mineralization effect corresponding to at least 65% of the re-mineralization effect of daily brushing of teeth with a new toothbrush,
f) optionally an anti-calculus effect corresponding to at least 25% of the anti-calculus effect of daily brushing of teeth with a new toothbrush, and
g) a substantially lower abrasive effect than the abrasive effect caused by daily brushing of teeth with a new toothbrush.

3. A chewing gum according to claim 2, wherein the whitening effect corresponds to at least 65%, preferably at least 85%, and more preferably at least 100%, of the whitening effect of daily brushing of teeth with a new toothbrush.

4. A chewing gum according to claim 2 or 3, wherein the anti-plaque effect corresponds to at least 65%, preferably at least 85%, and more preferably at least 100%, of the anti-plague effect of daily brushing of teeth with a new toothbrush.

5. A chewing gum according to one or more of claims 2 to 4, wherein the anti-gingivitis effect corresponds to at least 75%, preferably at least 85%, and more preferably at least 100%, of the anti-gingivitis effect of daily brushing of teeth with a new toothbrush.

6. A chewing gum according to one or more of claims 2 to 5, wherein the re-mineralization effect corresponds to at least 75%, preferably at least 85%, and more preferably at least 100%, of the re-mineralization effect of daily brushing of teeth with a new toothbrush.

7. A chewing gum according to one or more of claims 2 to 6, wherein the anti-calculus effect corresponds to at least 50%, preferably at least 65%, and more preferably at least 100%, of the anti-calculus effect of daily brushing of teeth with a new toothbrush.

8. A chewing gum according to one or more of claims 1 to 7, wherein pieces of the chewing gum are intended for chewing one, two or more times per day.

9. A chewing gum according to claim 1 or 2, wherein the individual piece of the chewing gum is intended for chewing for about 5 to about 20 minutes.

10. A chewing gum according to one or more of the preceding claims, wherein the chewing gum comprises one or more taste ingredients selected from sweeteners, high-potent sweeteners and/or flavours.

11. A chewing gum according to one or more of the preceding claims, wherein the chewing gum is manufactured from coherent gum.

12. A chewing gum according to one or more of the preceding claims 1-10, wherein the chewing gum is manufactured from compressed granules.

13. A chewing gum according to one or more of the preceding claims, wherein the chewing gum is layered.

14. A chewing gum according to claim 13, wherein at least one of the active ingredients is present in the chewing gum in only one of the layers.

15. A chewing gum according to claim 13 or 14, wherein two or more of the layers include active ingredients which are not present in all layers.

16. A chewing gum according to one or more of the claims 13-15, wherein the chewing gum comprises at least one barrier layer.

17. A chewing gum according to one or more of the preceding claims, wherein the chewing gum is coated.

18. A chewing gum according to claim 17, wherein at least one of the active ingredients is present in the coating.

19. A chewing gum according to one or more of the preceding claims, wherein said at least one anti-plaque agent constitute 0.01-70%, preferably 0.03-50%, more preferred 0.05-35% by weight of the chewing gum.

20. A chewing gum according to claim one or more of the preceding claims, wherein said at least one anti-gingivitis agent constitutes 0.01-20%, preferably 0.03-12% by weight of the chewing gum.

21. A chewing gum according to one or more of the preceding claims, wherein said at least one anti-calculus agent constitutes 0.01-20%, preferably 0.03-15 by weight of the chewing gum.

22. A chewing gum according to one or more of the preceding claims, wherein said at least one re-mineralization agent constitutes 0.01-20%, preferably 0.02-10 by weight of the chewing gum.

23. A chewing gum according to one or more of the preceding claims, wherein said at least one whitening agent constitutes 0.01-20%, preferably 0.03-12% by weight of the chewing gum.

24. A chewing gum according to one or more of the preceding claims, wherein said at least one fresh-breath agent constitutes 0.01-20%, preferably 0,02-8% by weight of the chewing gum.

25. A chewing gum according to one or more of the preceding claims, wherein the gum base further includes at least one antibacterial agent, said anti-bacterial agent preferably constituting about 0.4 to 7.5% of the chewing gum.

26. A chewing gum according to one or more of the preceding claims, the chewing gum being substantially free of abrasives.

27. A chewing gum according to one or more of the preceding claims, wherein the anti-calculus agents and/or the anti-gingivitis agents and/or the re-mineralization agents are free from metal ions.

28. A chewing gum according to one or more of the preceding claims, wherein said at least one whitening agent, said at least one fresh-breath agent, said at least one anti-plaque agent, and said at least one anti-gingivitis agent are mutually different substances.

29. A chewing gum according to one or more of the preceding claims, said chewing gum being intended for round the year daily use as the major tooth cleaning agent.

30. Use of a chewing gum according to one or more of the preceding claims as the major tooth cleaning agent for a period of at least one year.

31. Use of a chewing gum according to one or more of the preceding claims as a substitute to tooth brushing for a period of at least three months.
